# EUROPEAN PATENT SPECIFICATION

(11) **EP 3 378 512 B1**
(45) Date of publication and mention of the grant of the patent: **19.06.2024**
(21) Application number: 16866459.7
(22) Date of filing: 18.11.2016
(51) Int. Cl.: A61M 5/142

(54) **MEDICAL PUMP, METHOD FOR CONTROLLING MEDICAL PUMP, AND PROGRAM FOR CONTROLLING MEDICAL PUMP**
MEDIZINISCHE PUMPE, VERFAHREN ZUR STEUERUNG EINER MEDIZINISCHEN PUMPE UND PROGRAMM ZUR STEUERUNG EINER MEDIZINISCHEN PUMPE
POMPE MÉDICALE, PROCÉDÉ DE COMMANDE DE POMPE MÉDICALE, ET PROGRAMME DE COMMANDE DE POMPE MÉDICALE

(30) Priority: 20.11.2015 JP 2015227943
(43) Date of publication of application: 26.09.2018
(73) Proprietor: Terumo Kabushiki Kaisha, Tokyo 151-0072 (JP)
(72) Inventor: FUKUSHIMA, Eisuke, Ashigarakami-gun Kanagawa 259-0151 (JP); YOSHIMACHI, Daisuke, Ashigarakami-gun Kanagawa 259-0151 (JP); NOMURA, Takafumi, Ashigarakami-gun Kanagawa 259-0151 (JP)
(74) Representative: KIPA AB
(86) International application number: PCT/JP2016/084315
(87) International publication number: WO 2017/086459

(56) References cited:
- JP-A- S61 247 475
- JP-B2- 5 457 364
- US-A- 4 692 145
- US-A1- 2011 184 383
- US-A1- 2012 150 114

## Description

### Technical Field

The present invention relates to a medical pump for delivering a medication liquid or the like to a patient or the like, a medical pump control method, and a medical pump control program.

### Background Art

For example, medical pumps such as infusion pumps and syringe pumps are conventionally used to precisely control, for example, a dosage, an administration rate, or the like of a medication liquid when the medication liquid is administered to a patient (for example, Patent Literature 1) .

Such a medical pump is electrically driven, and thus, typically includes an "AC adapter cable" incorporating an "AC adapter" that converts an alternate current (AC) power supply supplied from an outlet to direct current to be applied for a medical pump.

In addition, the medical pump has a built-in battery so as to be able to operate without using the AC adapter cable.

### Citation List

### Patent Literature

Patent Literature 1: JP 2002-113099 A
US 2012/150114 discloses a battery management system for one or more batteries. Further relevant prior art can be found in US 4,692,145 A, and US 2011/0184383 A1.

### Summary of Invention

### Technical Problem

This, however, includes a problem of sudden stoppage of operation or the like in a case where both the AC adapter cable and a built-in battery fail. In addition, there is another problem that, in a case where a user operating a medical pump unplugs the AC adapter cable from the outlet or the like without knowing the failure or the like of the internal battery, the operation of the medical pump suddenly stops, for example, in a similar manner.

It is therefore an object of the present invention to provide a medical pump, a medical pump control method, and a medical pump control program capable of alerting in advance the user or the like to the stoppage or the like of the operation of the medical pump.

### Solution to Problem

The invention is defined by the claims.

The present invention is provided to achieve the above aims by a medical pump including: an external power acquisition unit that acquires power from the outside; two battery units capable of supplying power without intervention of the external power acquisition unit, wherein the two battery units include a first battery unit and a second battery unit, wherein the second battery unit is an auxiliary battery unit that supplies power in place of the first battery unit;
an AC power supply connection information processing unit, that determines whether the power acquisition unit is connected to a power supply; a first battery connection management unit and a second battery connection management unit, that detect whether the first and/or second battery is in a disconnected state; and an abnormality information storage unit that stores abnormality information of the external power acquisition unit and the two battery units, wherein the abnormality information includes that the external power acquisition unit is not connected to an outlet to acquire power from the outside, the first battery unit is disconnected, and the second battery unit is disconnected wherein when abnormality information, detecting disconnection of any two of the external power acquisition unit and the two battery units is stored in the abnormality information storage unit, power supply abnormality state information is output.

According to the above configuration, there are provided: an external power acquisition unit (for example, an AC power supply unit such as an AC adapter cable) that acquires power from the outside; and two battery units (for example, a main battery and a sub battery) capable of supplying power without intervention of the external power acquisition unit. With this configuration, in a case where there is a failure or the like in the external power acquisition unit and one of the two battery units, or where the use of the external power acquisition unit is stopped during failure of one of the battery units, the other battery unit supplies power, making it possible to maintain operation or the like of the medical pump, leading to prevention of an occurrence of a serious problem on a patient or the like using the medical pump.

Moreover, the above configuration outputs power supply abnormality state information (for example, power supply abnormality information) when abnormality information of any two of the external power acquisition unit and the two battery units is stored in the abnormality information storage unit.

With this configuration, it is possible to detect or the like an extremely critical power supply state that abnormality information is generated in two portions of the three, for example, and possible to notify the risk to the user before actual power supply stoppage, leading to prevention of the occurrence of a serious problem in a patient or the like using the medical pump.

Preferably, the two battery units include a first battery unit and a second battery unit, and a power exhaustion state in which the power stored in the first battery unit lowers to a predetermined level or below with difficulty in supplying a certain level of power is not included in the abnormality information.

According to the above configuration, the power exhaustion state in which the power stored in the first battery unit (for example, the main battery) lowers to a predetermined level or below with difficulty in supplying a certain level of power is not included in the abnormality information. With this configuration, it is possible to prevent an occurrence of a situation in which a simple "exhaustion state" that is not a failure or the like of the first battery unit itself is determined as "abnormality information" similar to a failure or the like causing hindrance of the use of the medical pump.

Preferably, first battery unit exhaustion state information representing an exhaustion state of the first battery unit and second battery unit exhaustion state information representing an exhaustion state of the second battery unit are stored, and when both of the first battery unit exhaustion state information and the second battery unit exhaustion state information are present, warning information that there is a need to continue acquisition of power from the external power acquisition unit is output.

According to the above configuration, when both the first battery unit exhaustion state information and the second battery unit exhaustion state information are present, warning information (for example, "Do not unplug the AC cable" or the like) that there is a need to continue acquisition of power from the external power acquisition unit is output.

That is, the first battery unit exhaustion state information is not included in the "abnormality information", and thus, when both the first battery unit exhaustion state information and the second battery unit exhaustion state information are present, that is, when the battery unit and the auxiliary battery unit both have difficulty in supplying the power, the power supply abnormality state information would not be output, and thus, there is a possibility that the user or the like would stop using the external power acquisition unit (for example, unplugs the AC adapter cable from the outlet).

This would cause power stoppage, leading to stoppage of operation of the medical pump.

To prevent this, the above-described configuration in this case outputs warning information that there is a need to continue acquisition of power from the external power acquisition unit, so as to enable the user to maintain the use of the external power acquisition unit to prevent sudden stop or the like of operation of the medical pump.

With this configuration, it is possible to prevent an occurrence of a serious problem in a patient or the like using the medical pump.

The second battery unit is an auxiliary battery unit that supplies power in place of the first battery unit.

The present invention is provided to achieve the above object by a method for controlling a medical pump including: an external power acquisition unit that acquires power from the outside; a battery unit capable of supplying power without intervention of the external power acquisition unit; an auxiliary battery unit that supplies power in place of the battery unit; and an abnormality information storage unit that stores abnormality information of the external power acquisition unit, the battery unit, and the auxiliary battery unit, in which when abnormality information of any two of the external power acquisition unit, the battery unit, and the auxiliary battery unit is stored in the abnormality information storage unit, power supply abnormality state information is output.

The present invention is provided to achieve the above object by a medical pump control program that causes a medical pump including: an external power acquisition unit that acquires power from the outside; a battery unit capable of supplying power without intervention of the external power acquisition unit; an auxiliary battery unit that supplies power in place of the battery unit; and an abnormality information storage unit that stores abnormality information of the external power acquisition unit, the battery unit, and the auxiliary battery unit, to execute a step of outputting power supply abnormality state information when abnormality information of any two of the external power acquisition unit, the battery unit, and the auxiliary battery unit is stored in the abnormality information storage unit.

### Advantageous Effects of Invention

As described above, according to the present invention, it is possible to provide a medical pump, a medical pump control method, and a medical pump control program capable of alerting in advance the user or the like to the stoppage of the operation of the medical pump, or the like.

### Brief Description of Drawings

Fig. 1 is a schematic diagram illustrating a main configuration of an infusion pump, as an exemplary medical pump.
Fig. 2 is a schematic illustration of a main configuration of a liquid delivery driving unit of the infusion pump illustrated in Fig. 1.
Fig. 3 is a schematic block diagram illustrating the main configuration of the infusion pump of Fig. 1.
Fig. 4 is a schematic block diagram illustrating a main configuration of a first variety of information storage unit in Fig. 3.
Fig. 5 is a schematic block diagram illustrating a main configuration of a second variety of information storage unit in Fig. 3.
Fig. 6 is a schematic block diagram illustrating a main configuration of a third variety of information storage unit in Fig. 3.
Fig. 7 is a schematic block diagram illustrating a main configuration of a fourth variety of information storage unit in Fig. 3.
Fig. 8 is a schematic flowchart illustrating a main operation example or the like of the infusion pump according to the present embodiment.
Fig. 9 is another schematic flowchart illustrating a main operation example or the like of the infusion pump according to the present embodiment.
Fig. 10 is another schematic flowchart illustrating a main operation example or the like of the infusion pump according to the present embodiment.
Fig. 11 is another schematic flowchart illustrating a main operation example or the like of the infusion pump according to the present embodiment.

### Description of Embodiments

Hereinafter, preferred embodiments of the present invention will be described in detail with reference to the accompanying drawings or the like.

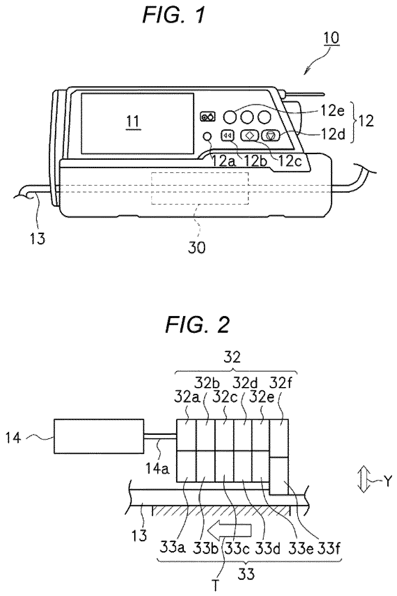

Fig. 1 is a schematic diagram illustrating a main configuration of an infusion pump 10 as an exemplary medical pump.

As illustrated in Fig. 1, the infusion pump 10 includes: a display 11 as a display unit that displays a variety of information; and various operation buttons 12.

For example, the various operation buttons 12 arranged include: a pilot lamp 12a that contains an LED and indicates operation states of the infusion pump 10 with light emission and display via a light diffusing agent; a fast delivery switch button 12b for injecting a medication liquid at an injection rate faster than a set flow rate (mL/h); a start switch button 12c; a stop switch button 12d; and a menu selection button 12e.

As illustrated in Fig. 1, the infusion pump 10 also includes a liquid delivery driving unit 30 that operates such as applying pressure on a tube 13 as an exemplary tube portion arranged inside the infusion pump 10, so as to deliver medication liquid therein.

The medication liquid delivered by the liquid delivery driving unit 30 is administered to a patient or the like with high accuracy.

Fig. 2 is a schematic illustration of a main configuration of the liquid delivery driving unit 30 of the infusion pump 10 illustrated in Fig. 1.

As illustrated in Fig. 2, the liquid delivery driving unit 30 includes a cam structure 32 and a finger structure 33, and liquid delivery of the medication liquid in the tube 13 is controlled with these structures.

That is, delivery of the medication liquid in the tube 13 is controlled with high accuracy by the operation of the cam structure 32.

Specifically, the cam structure 32 includes a plurality of cams, for example, six cams 32a to 32f, while the finger structure 33 includes six fingers 33a to 33f corresponding to the six cams 32a to 32f respectively.

The six cams 32a to 32f are arranged with a phase difference from each other, and the cam structure 32 is coupled to an output shaft 14a of a drive motor 14.

Specifically, with the drive and rotation of the drive motor 14 in Fig. 2, the plurality of fingers 33a to 33f is individually driven via the cams 32a to 32f respectively in the Y-direction in Fig. 2 so as to sequentially press an outer peripheral surface of the tube 13 in a T direction in Fig. 2 to deliver the medication liquid in the tube 13.

As described above, the liquid delivery driving unit 30 controls a peristaltic-like movement of the plurality of fingers 33a to 33f to sequentially move the fingers 33a to 33f forward/backward to move a blocking point within the tube 13 in the T direction as if waves propagate so as to squeeze the tube 13 to transfer the medication liquid.

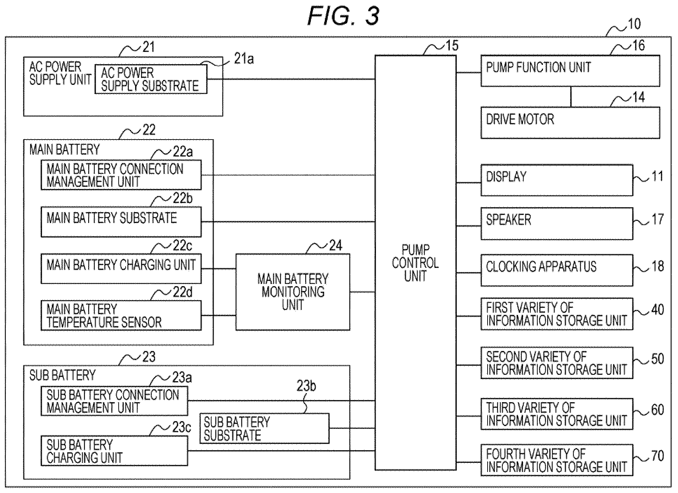

Fig. 3 is a schematic block diagram illustrating a main configuration of the infusion pump 10 of Fig. 1. As illustrated in Fig. 3, the infusion pump 10 includes a "pump control unit 15". The pump control unit 15 controls a "pump function unit 16" that controls the drive motor 14 or the like illustrated in Fig. 2.

Moreover, the pump control unit 15 also controls a "speaker 17" that output voice or the like and a "clocking apparatus 18" or the like, in addition to the display 11 illustrated in Fig. 1.

Furthermore, the pump control unit 15 controls: an AC power supply unit 21 as an exemplary external power acquisition unit; a main battery 22 as an exemplary first battery unit; a sub battery 23 as an exemplary second battery unit or an auxiliary battery unit; and a main battery monitoring unit 24, or the like.

As illustrated in Fig. 3, the pump control unit 15 also controls a "first variety of information storage unit 40", a "second variety of information storage unit 50", a "third variety of information storage unit 60" and a "fourth variety of information storage unit 70". These configurations will be described below.

Hereinafter, configurations of the AC power supply unit 21, the main battery 22, the sub battery 23, and the main battery monitoring unit 24 in Fig. 3 will be described.

First, the AC power supply unit 21 will be described. The AC power supply unit 21 includes an AC power supply substrate 21a and also includes an AC adapter cable connected to an outlet or the like in practice.

In addition, the main battery 22 is a battery that stores electricity inside, and is used as a power supply source when the AC adapter cable of the AC power supply unit 21 is not connected to an outlet, or the like. Moreover, the main battery 22 is rechargeable via the AC power supply unit 21.

Moreover, the main battery 22 includes a "main battery connection management unit 22a". The main battery connection management unit 22a can grasp whether the main battery 22 is in a disconnected state, or the like, due to line breakage, connector disengagement, or the like.

Furthermore, as illustrated in Fig. 3, the main battery 22 includes a "main battery substrate 22b", and further includes a "main battery charging unit 22c" that manages a charging state or the like of the main battery 22, and a "main battery temperature sensor 22d" that measures the temperature of the main battery 22.

Subsequently, the main battery monitoring unit 24 acquires "battery life determination data", "charge abnormality determination data", "battery remaining capacity data" or the like from the main battery charging unit 22c, while acquiring "temperature sensor data" from the "main battery temperature sensor 22d".

Details of the "battery life determination data", "charge abnormality determination data", "battery remaining capacity data" and "temperature sensor data" will be described below.

Next, the sub battery 23 will be described. The sub battery 23 is a battery used in place of the main battery 22 when the main battery 22 has difficulty in supplying power.

Similarly to the main battery 22, the sub battery 23 is rechargeable via the AC power supply unit 21 and includes a "sub battery connection management unit 23a" so as to be able to grasp whether the sub battery 23 is in a disconnected state, or the like, due to line breakage, connector disengagement, or the like.

The sub battery 23 also includes a "sub battery substrate 23b", and a "sub battery charging unit 23c" that manages the charging state or the like.

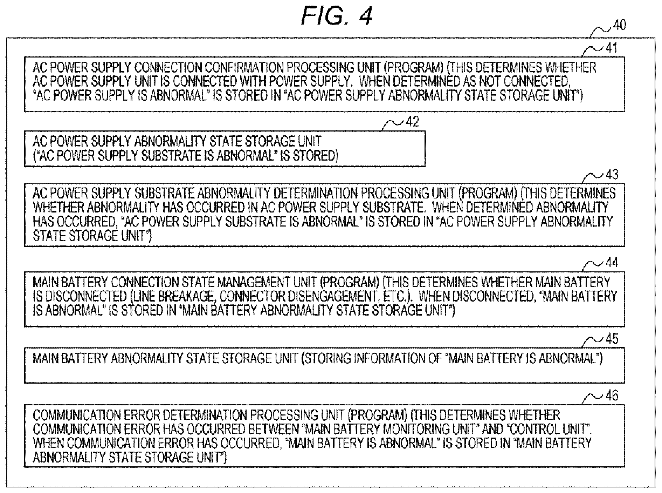

Figs. 4 to 7 are schematic block diagrams illustrating main configurations of the "first variety of information storage unit 40", the "second variety of information storage unit 50", the "third variety of information storage unit 60" and the "fourth variety of information storage unit 70" in Fig. 3. The specific configuration of these will be described below.

The infusion pump 10 illustrated in Fig. 1 has a computer including a central processing unit (CPU), a random access memory (RAM), and a read only memory (ROM) (these are not illustrated), being connected to each other via a bus.

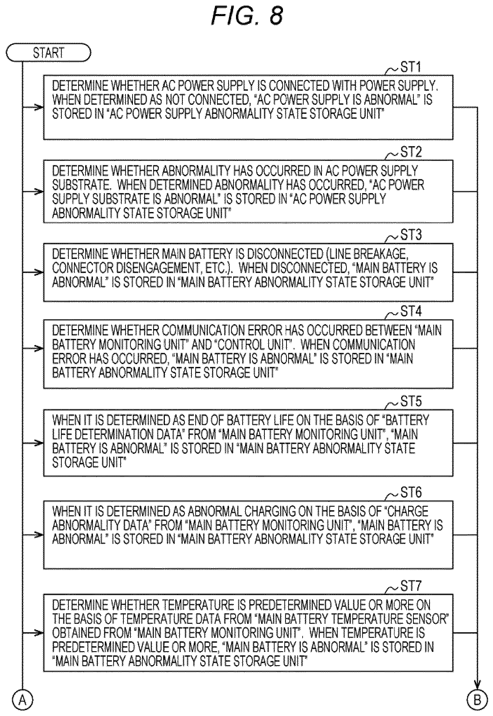

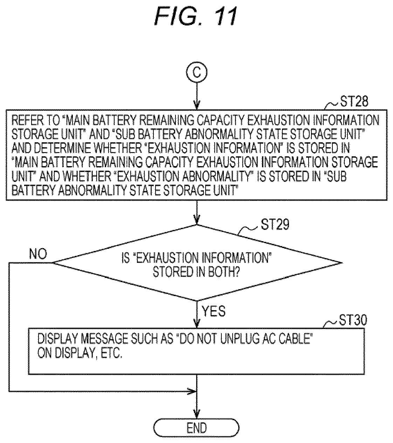

Figs. 8 to 11 are schematic flowcharts illustrating a main operation example or the like of the infusion pump 10 according to the present embodiment.

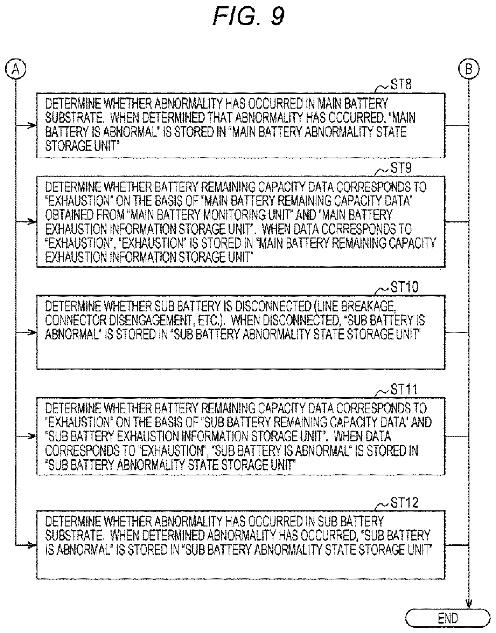

As illustrated in Figs. 8 and 9, the infusion pump 10 acquires information of the AC power supply unit 21, the main battery 22, the sub battery 23, or the like in Fig. 3 during operation (during liquid delivery). Details will be described below.

Steps of Figs. 8 and 9 are numbered for the sake of convenience, and these steps may be executed in order or simultaneously.

First, in step (hereinafter referred to as "ST") 1, the "AC power supply connection confirmation processing unit (program) 41" in Fig. 4 operates and determines whether the "AC power supply unit 21" is connected to the power supply. Specifically, in a case where the AC adapter cable is not connected to the outlet, it is determined as "abnormality", for example, and "AC power supply is abnormal" is stored in the "AC power supply abnormality state storage unit 42" as an exemplary abnormality information storage unit in Fig. 4.

That is, the infusion pump 10 uses the AC power supply unit 21 in normal use, having the main battery 22 as a supplementary power supply for the time when the AC power supply unit 21 is not available such as during movement and power failure. In this sense, the case where the AC adapter cable is not connected to an outlet is included in "abnormality".

Next, the processing proceeds to ST 2. In ST 2, "AC power supply substrate abnormality determination processing unit (program) 43" in Fig. 4 operates and determines whether an abnormality has occurred in the AC power supply substrate 21a. In a case where it is determined that the abnormality has occurred, "AC power supply substrate is abnormal" is stored in the "AC power supply abnormality state storage unit 42" in Fig. 4.

Next, processing proceeds to ST 3. In ST 3, the "main battery connection state management unit (program) 44" in Fig. 4 operates and determines whether the main battery 22 is disconnected, for example, whether line breakage or connector disengagement has occurred. In the case of disconnection, "main battery is abnormal" is stored in the "main battery abnormality state storage unit 45" in Fig. 4.

Next, the processing proceeds to ST 4. In ST 4, the "communication error determination processing unit (program) 46" in Fig. 4 operates and determines whether a communication error has occurred between the "main battery monitoring unit 24" and the "pump control unit 15" or the like in Fig. 3. In the case of occurrence of a communication error, "main battery is abnormal" is stored in the "main battery abnormality state storage unit 45" in Fig. 4.

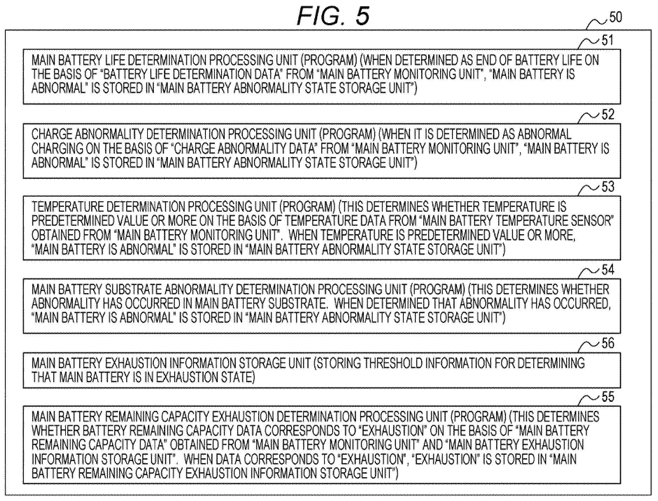

Next, the processing proceeds to ST 5. In ST 5, the "main battery life determination processing unit (program) 51" in Fig. 5 operates. In a case where it is determined as an end of battery life on the basis of "battery life determination data" such as data of the transition of the charge amount from the "main battery monitoring unit 24" in Fig. 3, for example, "main battery is abnormal" is stored in the "main battery abnormality state storage unit 45" in Fig. 4.

Next, the processing proceeds to ST 6. In ST 6, the "charge abnormality determination processing unit (program) 52" in Fig. 5 operates. In a case where it is determined as charge abnormality on the basis of "charge abnormality data" such as data of the transition of the charge amount from the "main battery monitoring unit 24" in Fig. 3, for example, "main battery is abnormal" is stored in the "main battery abnormality state storage unit 45" in Fig. 4.

Next, the processing proceeds to ST 7. In ST 7, the "temperature determination processing unit (program) 53" in Fig. 5 operates and determines whether the temperature is a predetermined value or more on the basis of temperature data from the "main battery temperature sensor" acquired from the "main battery monitoring unit 24" in Fig. 3. In a case where the temperature is the predetermined value or more, "main battery is abnormal" is stored in the "main battery abnormality state storage unit 45" in Fig. 4.

Then, the processing proceeds to ST8. In ST 8, the "main battery substrate abnormality determination processing unit (program) 54" in Fig. 5 operates and determines whether an abnormality has occurred in the main battery substrate 22b. In a case where it is determined that the abnormality has occurred, "main battery is abnormal" is stored in the "main battery abnormality state storage unit 45" in Fig. 4.

Next, the processing proceeds to ST 9. In ST 9, "main battery remaining capacity exhaustion determination processing unit (program) 55" in Fig. 5 operates and refers to "main battery remaining capacity data" acquired from the "main battery monitoring unit 24" in Fig. 3 and refers to the "main battery exhaustion information storage unit 56" in Fig. 5. The main battery exhaustion information storage unit 56 stores threshold information such as a numerical value for determining that the main battery 22 is in an exhaustion state.

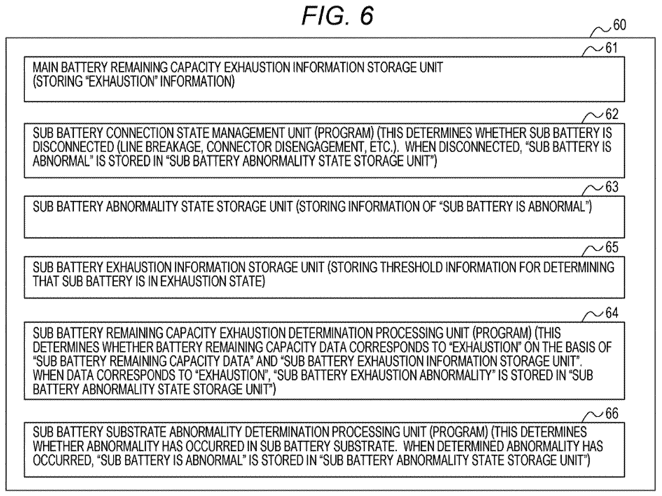

Accordingly, the main battery remaining capacity exhaustion determination processing unit (program) 55 determines whether the battery remaining capacity data corresponds to "exhaustion" on the basis of these. In a case where the data corresponds to "exhaustion", "exhaustion" information is stored in in the "main battery remaining capacity exhaustion information storage unit 61" in Fig. 6. The "exhaustion" information is exemplary first battery unit side exhaustion state information corresponding to an exemplary exhaustion state (power exhaustion state in which power stored in the battery unit lowers to a predetermined level or below with difficulty in supplying a certain level of power).

As described above, in the present embodiment, the power exhaustion state is not included in the abnormality information. With this configuration, it is possible to prevent an occurrence of a situation in which a simple "exhaustion state" that is not a failure or the like of the main battery 22 itself is determined as "abnormality information" similar to a failure or the like causing hindrance of the use of the infusion pump 10.

Then, the processing proceeds to ST10. In ST 10, the "sub battery connection state management unit (program) 62" in Fig. 6 operates and determines whether the "sub battery 23" in Fig. 3 is disconnected, for example, whether line breakage or connector disengagement has occurred. In the case of disconnection, "sub battery is abnormal" is stored in the "sub battery abnormality state storage unit 63" in Fig. 6.

Then, the processing proceeds to ST11. In ST11, the "sub battery remaining capacity exhaustion determination processing unit (program) 64" in Fig. 6 operates and refers to the "sub battery remaining battery remaining capacity data" from the "sub battery charging unit 23c" and refers to the "sub battery exhaustion information storage unit 65" in Fig. 6.

The sub battery exhaustion information storage unit 65 stores threshold information such as a numerical value for determining that the sub battery 23 is in the exhaustion state.

Accordingly, the sub battery remaining capacity exhaustion determination processing unit (program) 64 determines whether the battery remaining capacity data corresponds to "exhaustion" on the basis of these. When the data corresponds to "exhaustion", "sub battery is abnormal" is stored in the "sub battery abnormality state storage unit 63" in Fig. 6.

This "exhaustion" information is exemplary second battery unit side exhaustion state information.

In addition, this step also stores "exhaustion abnormality" as specific description of abnormality.

The sub battery 23 is intended to be used at emergency and thus is not assumed to be exhausted in normal use. Therefore, the case where the sub battery 23 is in the exhaustion state is determined as "abnormality", unlike the main battery 22.

Next, the processing proceeds to ST12. In ST 12, "sub battery substrate abnormality determination processing unit (program) 66" in Fig. 6 operates and determines whether an abnormality has occurred in the "sub battery substrate 23b" in Fig. 3. In a case where it is determined that the abnormality has occurred, "sub battery is abnormal" is stored in the "sub battery abnormality state storage unit 63" in Fig. 6.

The above is description of the infusion pump 10 that executes steps of acquisition, determination, and storage or the like of the information of the AC power supply unit 21, the main battery 22, the sub battery 23 or the like in Fig. 3 to be executed during operation of the pump (during liquid delivery). In addition to this, the following will describe steps of issuing warning or the like by the infusion pump 10 using such information.

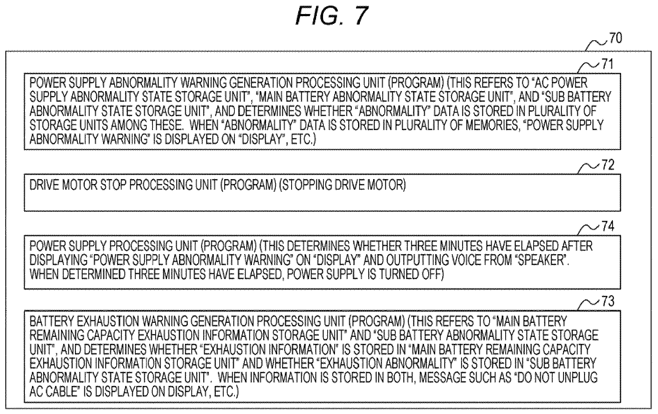

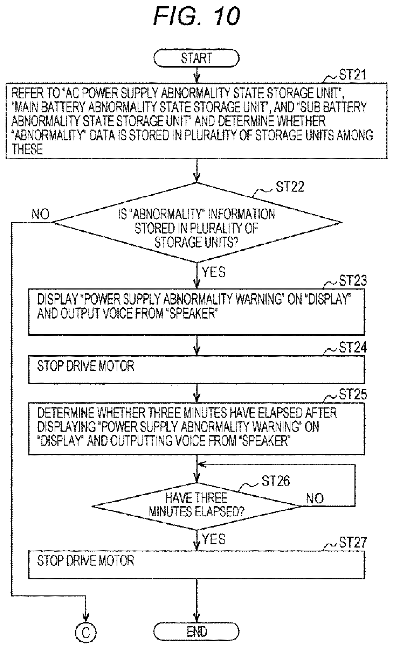

First, the processing proceeds to ST 21 of Fig. 10. In ST 21, the "power supply abnormality warning generation processing unit (program) 71" in Fig. 7 operates and refers to the "AC power supply abnormality state storage unit 42", the "main battery abnormality state storage unit 45" in Fig. 4 and the "sub battery abnormality state storage unit 63" in Fig. 6, and determines whether "abnormality" data is stored in a plurality of storage units among these storage units.

Then, ST 22 determines whether "abnormality" information is stored in a plurality of storage units, that is, whether "abnormality" data is stored in any two storage units among the "AC power supply abnormality state storage unit 42", the "main battery abnormality state storage unit 45", and the "sub battery abnormality state storage unit 63". When the data is stored, the processing proceeds to ST 23.

In ST 23, "power supply abnormality warning" is displayed on the "display 11" in Fig. 3 and the voice of power abnormality warning is output from the "speaker 17".

With this configuration, medical staff or the like as the user of the infusion pump 10 can quickly recognize the power supply abnormality of the infusion pump 10 and grasp the danger before stoppage of power supply to take necessary measures.

In addition, it is possible to prevent an occurrence of a serious problem in a patient or the like using the infusion pump 10.

Next, in ST 24, the "drive motor stop processing unit (program) 72" in Fig. 7 operates to stop the drive motor 14. Note that the stop of the drive motor 14 may be performed before the occurrence of a power supply abnormality warning or at the same time as the stop of the drive motor 14.

This makes it possible to quickly stop the liquid delivery in the abnormal power supply state and prevent an occurrence of a serious problem in a patient or the like using the infusion pump 10.

Note that unlike the conventional case, the present embodiment includes the sub battery 23 in addition to the AC power supply unit 21 and the main battery 22. Accordingly, even in a case where there is a failure or the like in the AC power supply unit 21 and the main battery 22, or in a case where the AC adapter cable of the AC power supply unit 21 is unplugged from the outlet when the main battery 22 is faulty or the like, the sub battery 23 supplies power.

This makes it possible to maintain the operation of the infusion pump 10 in which a power supply abnormality warning or the like occurs, leading to achievement of prevention of an occurrence of a serious problem in a patient or the like using the infusion pump 10.

Then, the processing proceeds to ST 25. In ST 25, the "power supply processing unit (program) 74" in Fig. 7 operates and determines whether three minutes have elapsed after displaying a "power supply abnormality warning" as exemplary power supply abnormality state information on the "display 11" and outputting voice from the "speaker 17".

Next, when it is determined that three minutes have elapsed in ST26, the processing proceeds to ST27, and the power is turned OFF.

With this configuration of stopping the operation of the infusion pump 10, it is possible to prevent the occurrence of a situation in which the infusion pump 10 suddenly stops due to insufficient power supply during liquid delivery.

In another case where "abnormality" information is not stored in the plurality of storage units in ST 22, the main battery 22 is configured so as not to include the power exhaustion state in the abnormality information, as described above.

For this reason, provided that the AC adapter cable is connected to the outlet, no abnormality is stored in the plurality of storage units and thus no "power supply abnormality warning" is output even when the power of the main battery 22 is in the exhaustion state and the sub battery 23 is also in the exhaustion state.

Still, when a user such as a nurse unplugs the AC adapter cable from the outlet, power supply is stopped to cause sudden stop of the infusion pump 10.

Therefore, in the present embodiment, in order to prevent such occurrence, the following steps are executed.

First, the processing proceeds to ST 28. In ST 28, the "battery exhaustion warning generation processing unit (program) 73" in Fig. 7 operates and refers to the "main battery remaining capacity exhaustion information storage unit 61" and the "sub battery abnormality state storage unit 63" in Fig. 6, and determines whether "exhaustion information" is stored in the "main battery remaining capacity exhaustion information storage unit 61" and whether "exhaustion abnormality" is stored in the "sub battery abnormality state storage unit 63".

Then, the processing proceeds to ST 29. ST 29 determines whether "exhaustion information" is stored in both the "main battery remaining capacity exhaustion information storage unit 61" and the "sub battery abnormality state storage unit 63", and when it is stored, processing proceeds to ST 30.

ST 30 displays a message such as "Do not unplug AC cable" or "Do not unplug AC cable due to insufficient charge" on the display 11 as warning information, for example.

The present embodiment is not limited to this and may use a step of first outputting a "power supply abnormality warning" to the display 11 and the speaker 17 and thereafter stopping the operation of the drive motor 14.

As described above, according to the present embodiment, since the warning information that there is a need to continue acquisition of power from the AC adapter cable is output, enabling the user to maintain the use of the AC adapter cable and enabling prevention of a sudden stop or the like of the operation of the infusion pump 10.

Accordingly, it is possible to prevent an occurrence of a serious problem in a patient or the like using the infusion pump 10.

The present invention is not limited to the above-described embodiment. While the present embodiment describes the infusion pump 10 as an exemplary medical pump, the present invention is not limited to this. It is allowable to use a "syringe pump" that delivers a medication liquid in a syringe by moving a syringe pusher.

Moreover, while the above-described embodiment is a case of using the main battery and the sub battery used when the sub battery has difficulty in supplying power, it is also allowable to configure such that two batteries operate in parallel to provide assistance at the time when the AC power supply is in an abnormal state, or when one of the two batteries fails, the other battery may supply power.

### Reference Signs List

10 Infusion pump
11 Display
12 Variety of operation buttons
12a Pilot lamp
12b Fast delivery switch button
12c Start switch button
12d Stop switch button
12e Menu selection button
13 Tube
14 Drive motor
14a Output shaft
15 Pump control unit
16 Pump function unit 16
17 Speaker
18 Clocking apparatus
21 AC power supply unit
21a AC power supply substrate
22 Main battery
22a Main battery connection management unit
22b Main battery substrate
22c Main battery charging unit
22d Main battery temperature sensor
23 Sub battery
23a Sub battery connection management unit
23b Sub battery substrate
23c Sub battery charging unit
24 Main battery monitoring unit
40 First variety of information storage unit
41 AC power supply connection confirmation processing unit (program)
42 AC power supply abnormality state storage unit
43 AC power supply substrate abnormality determination processing unit (program)
44 Main battery connection state management unit (program)
45 Main battery abnormality state storage unit
46 Communication error determination processing unit (program)
50 Second variety of information storage unit
51 Main battery life determination processing unit (program)
52 Charge abnormality determination processing unit (program)
53 Temperature determination processing unit (program)
54 Main battery substrate abnormality determination processing unit (program)
55 Main battery remaining capacity exhaustion determination processing unit (program)
56 Main battery exhaustion information storage unit
60 Third variety of information storage unit
61 Main battery remaining capacity exhaustion information storage unit
62 Sub battery connection state management unit (program)
63 Sub battery abnormality state storage unit
64 Sub battery remaining capacity exhaustion determination processing unit (program)
65 Sub battery exhaustion information storage unit
66 Sub battery substrate abnormality determination processing unit (program)
70 Fourth variety of information storage unit
71 Power supply abnormality warning generation processing unit (program)
72 Drive motor stop processing unit (program)
73 Battery exhaustion warning generation processing unit (program)
74 Power supply processing unit (program)

## Claims

1. A medical pump comprising:
an external power acquisition unit (21) that acquires power from the outside;
two battery units capable of supplying power without intervention of the external power acquisition unit, wherein the two battery units include a first battery unit (22) and a second battery unit (23), wherein the second battery unit (23) is an auxiliary battery unit that supplies power in place of the first battery unit (22);
an AC power supply connection information processing unit, that determines whether the power acquisition unit (21) is connected to a power supply;
a first battery connection management unit and a second battery connection management unit, that detect whether the first and/or second battery is in a disconnected state, respectively; and
an abnormality information storage unit (42, 45, 63) that stores abnormality information of the external power acquisition unit and the two battery units, **characterized in that**
the abnormality information includes that the external power acquisition unit (21) is not connected to an outlet to acquire power from the outside, the first battery unit (22) is disconnected, and the second battery unit (23) is disconnected;
wherein when the abnormality information, detecting disconnection of any two of the external power acquisition unit (21) and the two battery units (22, 23), is stored in the abnormality information storage unit (42, 45, 63), power supply abnormality state information is output.

2. The medical pump according to claim 1,
wherein a power exhaustion state in which the power stored in the first battery unit (22) lowers to a predetermined level or below with difficulty in supplying a certain level of power is not included in the abnormality information.

3. The medical pump according to claim 2,
wherein first battery unit exhaustion state information representing an exhaustion state of the first battery unit (22) and second battery unit exhaustion state information representing an exhaustion state of the second battery unit (23) are stored, and
when both of the first battery unit exhaustion state information and the second battery unit exhaustion state information are present, warning information that there is a need to continue acquisition of power from the external power acquisition unit (21) is output.

4. A method for controlling a medical pump that comprises
an external power acquisition unit (21) that acquires power from the outside;
two battery units capable of supplying power without intervention of the external power acquisition unit, wherein the two battery units include a first battery unit (22) and a second battery unit (23), wherein the second battery unit (23) is an auxiliary battery unit that supplies power in place of the first battery unit (22;
an AC power supply connection information processing unit, that determines whether the power acquisition unit (21) is connected to a power supply;
a first battery connection management unit and a second battery connection management unit, that detect whether the first and/or auxiliary battery unit is in a disconnected state, respectively; and
an abnormality information storage unit (42, 45, 63) that stores abnormality information of the external power acquisition unit (21), the battery unit (22), and the auxiliary battery unit (23), the method comprising:
determining, using the AC power supply connection information processing unit, the first battery connection management unit and the second battery connection management unit, whether the AC power supply is disconnected from an outlet to acquire power from the outside, the first battery unit (22) is disconnected, or the auxiliary battery unit (23) is disconnected; and
storing the disconnection information as abnormality information in the abnormality information storage unit (42, 45, 63);
wherein when abnormality information of any two of the external power acquisition unit (21), the battery unit (22), and the auxiliary battery unit (23) is stored in the abnormality information storage unit (42, 45, 63), power supply abnormality state information is output.

5. A medical pump control program that, when executed by a computer of the medical pump, causes the computer to carry out the method as set forth in claim 4.

## Patentansprüche

1. Eine medizinische Pumpe, bestehend aus:
einer externe Stromerfassungseinheit (21), die den Strom von außen bezieht;
zwei Batterieeinheiten, die in der Lage sind, ohne Eingriff der externen Stromerfassungseinheit Energie zu liefern, wobei die zwei Batterieeinheiten eine erste Batterieeinheit (22) und eine zweite Batterieeinheit (23) umfassen, wobei die zweite Batterieeinheit (23) eine Hilfsbatterieeinheit ist, die anstelle der ersten Batterieeinheit (22) Energie liefert;
einer AC-Stromversorgungs-Verbindungsinformations-Verarbeitungseinheit, die feststellt, ob die Stromerfassungseinheit (21) an eine Stromversorgung angeschlossen ist;
einer ersten Batterieverbindungs-Managementeinheit und einer zweiten Batterieverbindungsmanagementeinheit, die erkennt, ob die erste Batterie und/oder die zweite Batterie sich in einem nicht angeschlossenen Zustand befinden; und
einer Abnormalitätsinformations-Speichereinheit (42, 45, 63), die Abnormalitätsinformationen der externen Stromerfassungseinheit und der beiden Batterieeinheiten speichert, **dadurch gekennzeichnet, dass**
die Abnormitätsinformation beinhaltet, dass die externe Stromerfassungseinheit (21) nicht an eine Steckdose angeschlossen ist, um Energie von außen zu beziehen, die erste Batterieeinheit (22) wird abgeklemmt, und die zweite Batterieeinheit (23) wird abgeklemmt;
wobei, wenn die Abnormalitätsinformation, die eine Unterbrechung von zwei beliebigen der externen Stromerfassungseinheit (21) und der zwei Batterieeinheiten (22, 23) erfasst, in der Abnormalitätsinformations-Speichereinheit (42, 45, 63) gespeichert wird, einer Energieversorgungs-Abnormalitätszustandsinformation ausgegeben wird.

2. Die medizinische Pumpe nach Anspruch 1,
wobei ein Energieerschöpfungszustand, in dem die in der ersten Batterieeinheit (22) gespeicherte Energie auf ein vorbestimmtes Niveau oder darunter sinkt, wobei es schwierig ist, ein bestimmtes Energieniveau zu liefern, nicht in der Abnormalitätsinformation enthalten ist.

3. Die medizinische Pumpe nach Anspruch 2,
wobei die Information über den Erschöpfungszustand der ersten Batterieeinheit einen Erschöpfungszustand der ersten Batterieeinheit darstellt (22) und Informationen über den Erschöpfungszustand der zweiten Batterieeinheit, die einen Erschöpfungszustand der zweiten Batterieeinheit darstellen (23) gespeichert sind, und
wenn sowohl die Information über den Erschöpfungszustand der ersten Batterieeinheit als auch die Information über den Erschöpfungszustand der zweiten Batterieeinheit vorhanden sind, wird eine Warninformation ausgegeben, dass es notwendig ist, die Erfassung von Energie von der externen Stromerfassungseinheit (21) fortzusetzen.

4. Verfahren zur Steuerung einer medizinischen Pumpe, die eine externe Stromerfassungseinheit (21) umfasst, die Folgendes erfasst Strom von außen;
zwei Batterieeinheiten, die Strom liefern können, ohne Eingriff der externen Stromerfassungseinheit, wobei die zwei Batterieeinheiten eine erste Batterieeinheit (22) und eine zweite Batterieeinheit (23) umfassen, wobei die zweite Batterieeinheit (23) eine Hilfsbatterieeinheit ist, die anstelle der ersten Batterieeinheit (22) Enegie liefert;
einer AC-Stromversorgungs-Verbindungsinformations-Verarbeitungseinheit, die bestimmt, ob die Stromerfassungseinheit (21) an eine Stromversorgung angeschlossen ist;
eine erste Batterieverbindungs-Managementeinheit und eine zweite Batterieverbindungsmanagementeinheit, die erkennt, ob die erste Batterieeinheit und/oder die Hilfsbatterieeinheit sich in einem nicht angeschlossenen Zustand befinden; und
eine Abnormalitätsinformations-Speichereinheit (42, 45, 63), die Abnormalitätsinformationen der externen Stromerfassungseinheit (21), der Batterieeinheit (22) und der Hilfsbatterieeinheit (23) speichert, wobei das Verfahren umfasst:
Bestimmen, unter Verwendung der AC-Stromversorgungs-Verbindungsinformations-Verarbeitungseinheit, der ersten Batterieverbindungs-Managementeinheit und der zweiten Batterieverbindungs-Managementeinheit, ob die AC-Stromversorgung von einer Steckdose getrennt ist, um Energie von außen zu beziehen, die erste Batterieeinheit (22) getrennt ist oder die Hilfsbatterieeinheit (23) getrennt ist; und
Speicherung der Unterbrechungsinformationen als Anomalie Informationen in der Abnormalitätsinformations-Speichereinheit (42, 45, 63);
wobei, wenn Anomalieninformationen von zwei beliebigen Einheiten, nämlich der externen Stromerfassungseinheit (21), der Batterieeinheit (22) und der Hilfsbatterieeinheit (23), in der Abnormalitätsinformations-Speichereinheit (42, 45, 63) gespeichert sind, Informationen über den Anomalienzustand der Energieversorgung ausgegeben werden.

5. Ein medizinisches Pumpensteuerprogramm, das, wenn es von einem Computer der medizinischen Pumpe, veranlasst den Computer, die das in Anspruch 4 beschriebene Verfahren durchzuführen.

## Revendications

1. Pompe médicale comprenant :
une unité d'acquisition d'énergie externe (21) qui acquiert de l'énergie de l'extérieur ;
deux unités de batterie capables de fournir de l'énergie sans intervention de l'unité d'acquisition d'énergie externe, où les deux unités de batterie incluent une première unité de batterie (22) et une seconde unité de batterie (23), où la seconde unité de batterie (23) est une unité de batterie auxiliaire qui fournit de l'énergie à la place de la première unité de batterie (22) ;
une unité de traitement d'informations de branchement d'alimentation électrique en courant alternatif, qui détermine si l'unité d'acquisition d'énergie (21) est branchée à une alimentation électrique ;
une première unité de gestion de branchement de batterie et une seconde unité de gestion de branchement de batterie, qui détectent si la première batterie et/ou la seconde batterie est/sont dans un état débranché, respectivement ; et
une unité de stockage d'informations d'anomalie (42, 45, 63) qui stocke des informations d'anomalie de l'unité d'acquisition d'énergie externe et des deux unités de batterie, **caractérisée en ce que**
les informations d'anomalie incluent le fait que l'unité d'acquisition d'énergie externe (21) n'est pas branchée à une sortie pour acquérir de l'énergie de l'extérieur, que la première unité de batterie (22) est débranchée, et que la seconde unité de batterie (23) est débranchée ;
où lorsque les informations d'anomalie, détectant le débranchement de deux quelconques unités parmi l'unité d'acquisition d'énergie externe (21) et des unités de batterie (22, 23), sont stockées dans l'unité de stockage d'informations d'anomalie (42, 45, 63), une information d'état d'anomalie d'alimentation électrique est fournie.

2. Pompe médicale selon la revendication 1,
dans laquelle un état d'épuisement d'énergie dans lequel l'énergie stockée dans la première unité de batterie (22) descend à un niveau prédéterminé ou en dessous avec une difficulté à fournir un certain niveau d'énergie n'est pas inclus dans les informations d'anomalie.

3. Pompe médicale selon la revendication 2,
dans laquelle des informations d'état d'épuisement de première unité de batterie représentant un état d'épuisement de la première unité de batterie (22) et des informations d'état d'épuisement de seconde unité de batterie représentant un état d'épuisement de la seconde unité de batterie (23) sont stockées, et
lorsque à la fois les informations d'état d'épuisement de première unité de batterie et les informations d'état d'épuisement de seconde unité de batterie sont présentes, une information d'avertissement qu'il existe un besoin d'acquisition continue d'énergie depuis l'unité d'acquisition d'énergie externe (21) est fournie.

4. Procédé de commande d'une pompe médicale qui comprend
une unité d'acquisition d'énergie externe (21) qui acquiert de l'énergie de l'extérieur ;
deux unités de batterie capables de fournir de l'énergie sans intervention de l'unité d'acquisition d'énergie externe, où les deux unités de batterie incluent une première unité de batterie (22) et une seconde unité de batterie (23), où la seconde unité de batterie (23) est une unité de batterie auxiliaire qui fournit de l'énergie à la place de la première unité de batterie (22) ;
une unité de traitement d'informations de branchement d'alimentation électrique en courant alternatif, qui détermine si l'unité d'acquisition d'énergie (21) est branchée à une alimentation électrique ;
une première unité de gestion de branchement de batterie et une seconde unité de gestion de branchement de batterie, qui détectent si la première unité de batterie et/ou la seconde unité de batterie est/sont dans un état débranché, respectivement ; et
une unité de stockage d'informations d'anomalie (42, 45, 63) qui stocke des informations d'anomalie de l'unité d'acquisition d'énergie externe (21), de l'unité de batterie (22), et de l'unité de batterie auxiliaire (23), le procédé comprenant les étapes consistant à :
déterminer, en utilisant l'unité de traitement d'informations de branchement d'alimentation électrique en courant alternatif, la première unité de gestion de branchement de batterie et la seconde unité de gestion de branchement de batterie, si l'alimentation électrique en courant alternatif est débranchée d'une sortie pour acquérir de l'énergie de l'extérieur, que la première unité de batterie (22) est débranchée, ou que l'unité de batterie auxiliaire (23) est débranchée ; et
stocker les informations de débranchement en tant qu'informations d'anomalie dans l'unité de stockage d'informations d'anomalie (42, 45, 63) ;
où lorsque des informations d'anomalie de deux quelconques unités parmi l'unité d'acquisition d'énergie externe (21), l'unité de batterie (22), et l'unité de batterie auxiliaire (23) sont stockées dans l'unité de stockage d'informations d'anomalie (42, 45, 63), une information d'état d'anomalie d'alimentation électrique est fournie.

5. Programme de commande de pompe médicale qui, lorsqu'il est exécuté par un ordinateur de la pompe médicale, amène l'ordinateur à réaliser le procédé selon la revendication 4.
